**Europäisches Patentamt**

(19) **European Patent Office**

**Office européen des brevets**

(11) Publication number : **0 329 977 B1**

(12) **EUROPEAN PATENT SPECIFICATION**

(45) Date of publication of patent specification :
01.04.92 Bulletin 92/14

(51) Int. Cl.$^5$ : **A61K 47/26, A61K 9/20**

(21) Application number : **89101491.2**

(22) Date of filing : **28.01.89**

(54) **A xylitol-based binding and diluting agent and a process for the production thereof.**

(30) Priority : **25.02.88 FI 880892**

(43) Date of publication of application :
**30.08.89 Bulletin 89/35**

(45) Publication of the grant of the patent :
**01.04.92 Bulletin 92/14**

(84) Designated Contracting States :
**AT BE CH DE ES FR GB GR IT LI LU NL SE**

(56) References cited :
**EP-A- 0 193 651**
**FR-A- 2 336 123**
**FR-A- 2 380 777**

(73) Proprietor : **XYROFIN OY**
**Kyllikinportti 2**
**SF-00240 Helsinki (FI)**

(72) Inventor : **Virtanen, Jouko**
**Niittypolku 22**
**SF-02460 Kantvik (FI)**
Inventor : **Mäkelä, Matti**
**Harju 1 A 6**
**SF-02460 Kantvik (FI)**

(74) Representative : **Masch, Karl Gerhard et al**
**Patentanwälte Andrejewski, Honke & Partner**
**Theaterplatz 3 Postfach 10 02 54**
**W-4300 Essen 1 (DE)**

EP 0 329 977 B1

## Description

This invention relates to a binding and diluting agent suited for use in the production of tablets by direct compression technique and comprising mainly xylitol and a small amount of sorbitol, and to a process for the production thereof.

The direct compression technique has become increasingly popular in tablet manufacturing both in pharmaceutical and food industries, and binding and diluting agents suited for direct compression have been developed. An ideal binding and diluting agent also functions in the tablet either as an active ingredient or as an agent improving flavor or other such properties.

Sweet carbohydrates such as sugars and sugar alcohols are well suited for use as binding and diluting agents. A problem therewith is that crystalline or powdered sugars and sugar alcohols as such are poorly suited for direct compression. Therefore, granulated products have been developed for use in direct compression. In pharmaceutical and food industries, these products can be regarded as semi-finished products which are utilized as raw materials in the proper tabletting technique.

A good directly compressible material should be free-flowing; it should not get cloddy; and it should form firm tablets with a reasonable compression force. Many crystalline substances are not tabletable as such. Powders prepared from crystalline sugars and sugar alcohols are not suitable for use in direct compression as such, since they have a very poor flowability.

Commercial binding and diluting agents include "Emdex", an agglomerated dextrose; "DiPac", an agglomerated sucrose containing dextrines; "Starch 1500", a pregelatinized directly compressible starch; and mannitol. Finnish Patent Application 854,885 discloses a fructose-based binding and diluting agent, a fructose agglomerate, suited for use in direct compression. U.S. Patent Specification 4,352,821 discloses a product which is formed of fructose and salt. U.S. Patent Specification 4,159,345 discloses an excipient prepared from microcrystalline cellulose.

Having a pleasant flavour, xylitol is a carbohydrate well suited for the preparation of tablets. Xylitol is also significant as an anti-cariogenic substance. However, the use of xylitol in the direct compression technique has proved difficult. When compressed, crystalline xylitol does not form sufficiently firm and nevertheless easily soluble tablets. Powdered xylitol has an extremely poor flowability and a tendency to stick to the mould in the tablet machine.

Tablets can be formed of xylitol by means of the conventional wet granulation process with gelatin or starch as an additive. Finnish Patent Specification 61,392 discloses a process for the preparation of a tablet containing xylitol with the direct compression technique. In this process, 30 to 60% by weight of xylitol and 60 to 30% by weight of sorbitol and a small amount of flavoring and lubricating agents are dry mixed, and the resultant mixture is compressed to tablets. In the examples of the patent specification, the ratio of the amount of xylitol to the amount of sorbitol is 1:1 and about 0.43:1 (256:597). As a consequence, this process enables only a partial utilization of the anti-cariogenic influence and the advantageous flavoring properties of xylitol, because a major part of xylitol is replaced with sorbitol.

The object of the present invention is to provide a substantially xylitol-based product the flowability and mouldability of which are suitable for the production of tablets by direct compression technique.

This object is achieved by means of a binding and diluting agent according to the invention which is characterized in that it comprises freely flowing granules the average particle size of which varies within the range from 0.1 to 1 mm and which contain 94 to 98% by weight of xylitol; 1 to 5% by weight of sorbitol; 0 to 2% by weight of other polyols; and less than 1.0% by weight of water; and that the -bulk density thereof (tapped density, TD) varies from 0.7 to 0.8 g/cm³. This binding and diluting agent is a granulated product which has a good flowability and which is prepared according to the invention by agglomerating crystalline xylitol powdered to a small particle size by means of sorbitol syrup by simultaneously mixing the xylitol at a great speed, whereby a granular product is obtained; and by drying the granules to a water content less than 1.0%, whereby the amount of the sorbitol syrup is selected so that the obtained granular product contains 94 to 98% by weight of xylitol; 1 to 5% by weight of sorbitol; and 0 to 2% by weight of other polyols.

The flowability of the binding and diluting agent can be measured by allowing a sample (200 g) to flow through a hopper (runner pipe dimensions: diameter 8 mm and length 25 mm) on to a balance connected to a recording device. The flowability (s/100 g) of the substance can thus be calculated from the curve so obtained. The flowability should be better than 15 s/100 g. Another procedure for evaluating flowability is the determination of angle of repose: a sample (50 g) is passed slowly through a hopper on to a paper, and the angle defined between the paper and the formed hill is measured. This angle should not exceed 32°.

The bulk density of the substance can be measured by weighing a sample of 300 g into a measuring cylinder. The sample is poured carefully into the cylinder; the sample is weighed accurately; and its volume is recorded. The loose density (LD) can be calculated from this data. Thereafter the sample is vibrated at an amplitude of 1.5 mm, until it does no more pack to a smaller volume. The volume is recorded, and the bulk density (tapped density, TD) is calculated.

Xylitol used as raw material in the production of the binding and diluting agent according to the invention has a purity exceeding 95% by weight, and has been ground to an average particle size of 0.01 to 0.10 mm by means of a suitable mill, such as a hammer mill common in sugar industry to produce icing sugar.

Sorbitol is added to the xylitol at the granulation stage in the form of sorbitol syrup which may be e.g. hydrated starch syrup containing 50 to 90% by weight of sorbitol on dry substance, the rest consisting of higher sugar alcohols. Pure dissolved sorbitol is also suitable for use. Sorbitol syrup is diluted before granulation so that the dry substance content of the solution suits the granulation device. The nozzle structure of the Schugi device, for instance, requires a dry content below 50% by weight. The dry substance content of a commercial sorbitol syrup generally varies from 69 to 71% by weight. The dilution is preferably carried out by water, though a mixture of water and ethanol is also possible. The use of ethanol, however, is restricted by the poor solubility of sorbitol in ethanol.

The binding and diluting agent according to the invention is prepared by granulating the ground xylitol together with a small amount of sorbitol syrup by means of a suitable granulation device. The product is dried rapidly in a fluidized bed, for instance. There are several suitable granulation devices on the market. The dryer may be separate, or the drying may be carried out in a granulator, depending on the type of device. In the granulation device, the ground xylitol and the sorbitol syrup added evenly thereto are brought into a rapid movement which effects the agglomeration of the substance together with a small amount of syrup. The grain size can be adjusted by the mixture ratios and the mixing efficiency.

The granulated product is dried rapidly e.g. in a fluidized bed by means of dry air so that the final moisture content is below 1% by weight, preferably below 0.5% by weight. A suitable particle size is 0.1 to 1 mm in average, whereby about 99% of the granules is within this range. As used herein the term "dry air" refers to air with a water content not more than 7.5 grams of water per cubic meter of air, measured at 20 °C.

The product obtained according to the invention is a freely moving granulated binding and diluting agent which has excellent compressing properties and which withstands storing without getting cloddy. Its properties are preferably as follows:

| | |
|---|---|
| Moisture content | below 0.5% by weight |
| Average grain size | 0.1 to 0.6 mm |
| Xylitol | 95 to 98% by weight |
| Sorbitol | 2 to 3% by weight |
| Other polyols | 0 to 2% by weight |
| Bulk density (TD) | 0.7 to 0.8 g/cm$^3$ |
| Flowability | < 15 s/100 g (a hopper with a pipe diameter of 8 mm and a pipe length of 25 mm), or flow angle < 32° |

The details of the invention will be described in the following examples, which are merely intended to illustrate the invention and are not to be considered to limit the scope of the invention.

Example 1

Production of xylitol powder

Crystalline xylitol (purity over 95% by weight) was ground by means of a turbo mill (Bauermeister, manufacturer Gebr. Bauermeister & Co., Hamburg, West-Germany) to an average particle size of 0.07 mm. Over 50% of the particles was within the range from 0.02 to 0.10 mm, and the powder did not contain any particles exceeding 0.125 mm, nor a dusty fraction.

Example 2

Granulation of xylitol powder

A xylitol powder produced according to Example 1 and a 40% by weight sorbitol syrup solution (containing 34% by weight of sorbitol, and less than 5.7% of other polyols) were introduced into a granulator (Schugi, manufacturer Schugi, BV, Lelystad, Holland) at a speed of 800 kg/hour (powder) and 50 1/hour (syrup solution) at a temperature of 60°C. The spraying pressure was 2 bar and the rotative velocity 3,000 r/min, whereby grains having an average diameter of 0.42 mm were obtained (over 50% within the range from 0.2 to 0.6 mm). The resultant grains were dried with a fluidized-bed dryer (Schugi, manufacturer Schugi, BV, Lelystad, Holland). Granulate was fed into the dryer at a rate of 800 kg/h, and 10,000 m$^3$/hour of dry air was introduced therein.

The temperature of the drying air in the first quarter of the dryer was 45°C and in the second quarter 35°C; in the last two quarters of the dryer the temperature was room temperature, i.e. 20 to 25°C. The moisture content of the product was 0.3%, and the bulk density (TD) 0.74 g/ml.

The composition of the product was as follows:

```
Xylitol                    97%
Sorbitol                    2%
Other polyols       approx. 1%
Moisture                  0.3%
                          100%
```

The flowability of the granulate was extremely good (flow rate 12 s/100 g; flow angle 30°), and the properties of the granulate did not change during storage (75 days, 18°C).

Example 3

Compression of tablets

The granulate produced according to Example 2 was compressed to tablets by means of an eccentric press (Korsch EK-O/DMS; manufacturer Korsch OHG Maschinenfabrik, Berlin, West-Germany). Magnesium stearate (1%) was used as an additive. The diameter of the tablet was 11 mm and weight 500 mg. The crushing strength of tablets manufactured by different compression forces was determined in accordance with the instructions of European Pharmacopea. The results are shown in Table 1.

```
Table 1
```

| Compression force | Crushing strength |
|---|---|
| 5 kN | 46 N |
| 10 kN | 66 N |
| 15 kN | 88 N |
| 20 kN | 108 N |

Example 4

Compression of tablets

A granulate produced according to Example 2 was compressed to double-convex tablets by means of a rotation machine (Manesty D 3; manufacturer, Manesty Machines, Liverpool, England). Magnesium stearate (1%) was used as an additive. The diameter of the tablet was 15 mm and weight 907 mg. The crushing strength of the tablet (European Pharmacopea) was 127 N; the weight loss in friability tests was 0.71%.

**Claims**

1. A mainly xylitol-based binding and diluting agent suited for use in the production of direct compression tablets, **characterized** in that it comprises freely flowing granules the average particle size of which varries within the range from 0.1 to 1 mm and which contain 94 to 98% by weight of xylitol; 1 to 5% by weight of sorbitol; 0 to 2% by weight of other polyols; and less than 1.0% by weight of water; and that the bulk density thereof (tapped density, TD) varies from 0.7 to 0.8 g/cm$^3$.

2. A binding and diluting agent according to claim 1, **characterized** in that it contains 95 to 98% by weight of xylitol; 1.5 to 3.5% by weight of sorbitol; 0 to 1% by weight of other polyols; and less than 0.5% by weight of water; and that the flowability thereof is better than 15 s/100 g determined by allowing a sample of 200 g to flow through a hopper with a runner pipe diameter of 8 mm and length of 25 mm on to a balance connected to a recording device and calculating the flowability in s/100 g from the curve so obtained.

3. A process for the production of a xylitol-based binding and diluting agent according to claim 1 which is suited for use in the production of tablets by direct compression technique, **characterized** by the steps of agglomerating crystalline xylitol ground to a small particle size by means of sorbitol syrup simultaneously mixing the xylitol at a great speed, whereby a granular product is obtained; and drying the granules by means of dry air to a water content less than 1.0%, whereby the amount of sorbitol syrup to be used is selected so that the obtained granular product contains 94 to 98% by weight of xylitol; 1 to 5% weight of sorbitol; and 0 to 2% by weight of other polyols.

4. A process according to claim 3, **characterized** in that the amount of sorbitol syrup used in the agglomeration is 3 to 8% by weight calculated on the amount of the xylitol powder.

5. A process accordint to claim 3, **characterized** in that the sorbitol syrup contains less than 45, preferably 40% by weight of dry substance, of which more than 50, preferably more than 85% by weight is sorbitol.

6. A process according to claim 3, **characterized** in that the average particle size of the xylitol ground to a small grain size is 0.01 to 0.10 mm, whereby at least 50% of the particles are within said range.

7. A process accordint to claim 6, **characterized** in that the average particle size of the xylitol ground to a small grain size is 0.07 mm, at least 50% of the particles being within the range from 0.02 to 0.10 mm.


## Patentansprüche

1. Binde- und Verdünnungsmittel hauptsächlich auf Xylitbasis für die Herstellung von direkt verpreßten Tabletten, dadurch gekennzeichnet, daß es frei fließende Körnchen umfaßt, deren durchschnittliche Teilchengröße im Bereich von 0,1 bis 1 mm liegt, und welche 94 bis 98 Gew.-% Xylit, 1 bis 5 Gew.-% Sorbit, 0 bis 2 Gew.-% andere Polyole und weniger als 1,0 Gew.-% Wasser enthalten; und daß deren Schüttdichte (Klopfdichte, TD) von 0,7 bis 0,8 g/cm³ variiert.

2. Binde- und Verdünnungsmittel nach Anspruch 1, dadurch gekennzeichnet, daß es 95 bis 98 Gew.-% Xylit, 1,5 bis 3,5 Gew.-% Sorbit, 0 bis 1 Gew.-% andere Polyole und weniger als 0,5 Gew.-% Wasser enthält; und daß dessen Fließfähigkeit besser als 15 s/100 g ist, wobei die Fließfähigkeit bestimmt wird, indem eine Probe von 200 g durch einen Trichter mit einem Trichterrohrdurchmesser von 8 mm und einer -länge von 25 mm auf eine Waage fließen gelassen wird, die an ein Aufzeichnungsgerät angeschlossen ist, und indem die Fließfähigkeit in s/100 g aus der so erhaltenen Kurve berechnet wird.

3. Verfahren zur Herstellung eines Binde- und Verdünnungsmittels auf Xylitbasis nach Anspruch 1, das für die Herstellung von Tabletten durch Direktpressen geeignet ist, gekennzeichnet durch die Stufen des Agglomerierens von kristallinem auf kleine Teilchengrößen gemahlenem Xylit mittels Sorbitsirup unter gleichzeitigem Mischen des Xylits mit hoher Geschwindigkeit, wodurch ein körniges Produkt erhalten wird, und des Trocknens der Körnchen mittels trockener Luft auf einen Wassergehalt von weniger 1,0 %, wobei die Menge an verwendetem Sorbit so gewählt wird, daß das erhaltene körnige Produkt 94 bis 98 Gew.-% Xylit, 1 bis 5 Gew.-% Sorbit und 0 bis 2 Gew.-% andere Polyole enthält.

4. Verfahren nach Anspruch 3, dadurch gekennzeichnet, daß die beim Agglomerieren verwendete Menge an Sorbit 3 bis 8 Gew.-%, bezogen auf die Menge an Xylitpulver, ist.

5. Verfahren nach Anspruch 3, dadurch gekennzeichnet, daß der Sorbitsirup weniger als 45, vorzugsweise 40, Gew.-% Trockensubstanz enthält, von der mehr als 50, vorzugsweise mehr als 85, Gew.-% Sorbit ist.

6. Verfahren nach Anspruch 3, dadurch gekennzeichnete, daß die durchschnittliche Teilchengröße des auf eine kleine Korngröße gemahlenen Xylits 0,01 bis 0,10 mm ist, wobei mindestens 50 % der Teilchen in diesem Bereich liegen.

7. Verfahren nach Anspruch 6, dadurch gekennzeichnet, daß die durchschnittliche Teilchengröße des auf eine kleine Korngröße gemahlenen Xylits 0,07 mm ist, mindestens 5 % der Teilchen im Bereich von 0,02 bis 0,10 mm liegt.


## Revendications

1. Agent liant et diluant principalement à base de xylitol, approprié à l'emploi dans la production de comprimés par compression directe, caractérisé en ce qu'il comprend des granules coulants dont la granulométrie moyenne varie dans la gamme de 0,1 à 1 mm et contenant 94 à 98 % en poids de xylitol ; 1 à 5 % en poids de sorbitol ; 0 à 2 % en poids d'autres polyols ; et moins de 1,0 % en poids d'eau ; et en ce que sa masse volumique apparente (masse volumique apparente après tassement, MVT) varie de 0,7 à 0,8 g/cm³.

2. Agent liant et diluant selon la revendication 1, caractérisé en ce qu'il contient 95 à 98 % en poids de xylitol ; 1,5 à 3,5 % en poids de sorbitol ; 0 à 1 % en poids d'autres polyols et moins de 0,5 % en poids d'eau ; et en ce que sa fluidité, déterminée en laissant un échantillon de 200 g s'écouler à travers une trémie avec un tube d'écoulement ayant un diamètre de 8 mm et une longueur de 25 mm sur une balance raccordée à un dispositif d'enregistrement et calcul de la fluidité en s/100 g à partir de la courbe ainsi obtenue, est meilleure que 15 s/100 g.

3. Procédé pour préparer un agent liant et diluant à base de xylitol selon la revendication 1, convenant à l'emploi dans la production de comprimés selon la technique de compression directe, caractérisé par les étapes d'agglomération de xylitol cristallin, broyé à une granulométrie fine, à l'aide d'un sirop de sorbitol, avec mélange simultané du xylitol à grande vitesse pour obtenir un produit granulaire ; et de séchage des granules avec de l'air sec à une teneur en eau inférieure à 1,0 %, la quantité de sirop de sorbitol utilisée étant choisie de telle sorte que le produit granulaire obtenu contienne 94 à 98 % en poids de xylitol ; 1 à 5 % en poids de sorbitol ; et 0 à 2 % en poids d'autres polyols.

4. Procédé selon la revendication 3, caractérisé en ce que la quantité de sirop de sorbitol utilisée dans l'agglomération est de 3 à 8 % en poids, calculée relativement à la quantité de poudre de Xylitol.

5. Procédé selon la revendication 3, caractérisé en ce que le sirop de sorbitol contient moins de 45 et de préférence 40 % en poids de matières sèches dont plus de 50 et de préférence plus de 85 % en poids sont constitués de sorbitol.

6. Procédé selon la revendication 3, caractérisé en ce que la granulométrie moyenne du xylitol, broyé à une granulométrie fine, est de 0,01 à 0,10 mm, au moins 50 % des particules se situant dans ladite gamme.

7. Procédé selon la revendication 6, caractérisé en ce que la granulométrie moyenne du xylitol, broyé à une granulométrie fine, est de 0,07 mm, au moins 50 % des particules se situant dans la gamme de 0,02 à 0,10 mm.